# EUROPÄISCHE PATENTSCHRIFT

(11) **EP 4 487 897 B1**
(45) Veröffentlichungstag und Bekanntmachung des Hinweises auf die Patenterteilung: **22.10.2025**
(21) Anmeldenummer: 24169212.8
(22) Anmeldetag: 09.04.2024
(51) Int. Cl.: A61M 37/00, A61M 25/06

(54) **IMPLANTATSPRITZE**
IMPLANT SYRINGE
SERINGUE POUR IMPLANT

(30) Priorität: 04.07.2023 DE 102023117652
(43) Veröffentlichungstag der Anmeldung: 08.01.2025
(73) Patentinhaber: Gaplast GmbH, 82442 Altenau (DE)
(72) Erfinder: Kneer, Stephan, 82490 Farchant (DE); Kneer, Roland, 82490 Farchant (DE); Koller, Walter, 82497 Unterammergau (DE)
(74) Vertreter: Flosdorff, Jürgen

(56) Entgegenhaltungen:
- WO-A1-2023/011913
- DE-A1- 102017 007 893
- DE-A1- 102020 122 654

## Beschreibung

Die Erfindung betrifft eine Implantatspritze, die dazu verwendet wird, ein strangförmiges Feststoffimplantat mit einem Langzeitwirkstoff in den Körper eines Patienten abzugeben. Meist wird das Langzeitpräparat in die Bauchdecke eines Patienten gelegt, in die zuvor mittels einer Kanüle ein Aufnahmekanal für das Feststoffimplantat eingestochen wurde.

Die Erfindung geht von einer Implantatspritze aus, die folgendes Merkmale enthält: Eine Kanüle, ein Depot-Element zur Aufnahme eines Feststoffimplantats, eine Außenhülse mit einem radial überstehenden vorderen Griffabschnitt, mit dem die Kanüle gemeinsam mit dem Depot-Element und der Außenhülse axial bewegbar ist, ferner eine Innenhülse, auf der die Außenhülse sitzt und in der die Kanüle mit dem Depot-Element verschiebbar ist, wobei die Innenhülse axiale Schlitze aufweist, die radiale Stege der Außenhülse durchgreifen, wobei die Implantatspritze ferner einen stabförmigen Kolben aufweist, der an einem rückwärtigen Griffabschnitt befestigt ist und teilweise von einer hinteren Hülse umgeben ist, die verschieblich in die Innenhülse eingreifen kann, wobei der stabförmige Kolben durch das Depot-Element in die Kanüle soweit vorschiebbar ist, dass zwischen der Spitze der Kanüle und dem Kopfende des Kolbens ein Abstand verbleibt, der etwa gleich der Länge des abzugebenden Feststoffimplantats ist, wobei die Außenhülse in der vorgeschobenen Ausgangsstellung bei freiliegender Kanüle durch eine Blockiereinrichtung der Innenhülse lösbar arretiert ist und die Blockiereinrichtung beim Vorschub der hinteren Hülse in ihre Endstellung in die Freigabestellung bewegt wird, wodurch die Außenhülse mittels des vorderen Griffabschnitts auf der Innenhülse soweit zurückziehbar ist, dass die Kanüle vollständig in die Innenhülse eintritt.

Eine derartige Implantatspritze ist in der DE 10 2017 007 893 A1 offenbart. Bei dieser Implantatspritze ist die Außenhülse durch radiale Stege einstückig mit einer Nadelhalterung verbunden, in deren Rückseite das Depot-Element, das das Feststoffimplantat aufnimmt, eingefügt wird. Nach der Montage von Innenhülse und Außenhülse wird in eine vordere Öffnung der Nadelhalterung die Kanüle eingeklebt. Das Feststoffimplantat wird durch die offene Rückseite der Innenhülse in das Depot-Element eingesetzt und durch den stabförmigen Kolben in dem Depot-Element in die leicht eingeklemmte Ausgangsposition vorgeschoben. Bei der Anwendung der Implantatspritze zur Abgabe des Feststoffimplantats an einen Patienten wird dann der hintere Griffabschnitt mit dem Kolben vorgeschoben, bis das Feststoffimplantat bis zur Spitze der Kanüle vorgeschoben ist, woraufhin die in dieser Position freigegebene Außenhülse mit der Kanüle und dem Depot-Element unter Zurücklassung des Feststoffimplantats im Körper des Patienten zurückgezogen wird.

Aus der DE 10 2020 122 654 A1 ist eine Implantatspritze bekannt, bei der die Kanüle mit dem Depot-Element zu einer separaten, d.h. von der übrigen Implantatspritze getrennten Kanüleneinheit fest verbunden ist, wodurch das Einsetzen eines Feststoffimplantats in das Depot-Element erheblich vereinfacht ist. Die Erfindung sieht ferner vor, dass die Stege der Außenhülse an ihren radial inneren Enden an einem ringförmigen Aufnahmeteil angesetzt sind, das sich innerhalb der Innenhülse befindet, und dass die Kanüleneinheit mit in das Depot-Element eingesetztem Feststoff-implantat in das Aufnahmeteil einsteckbar und darin arretierbar ist.

Bei diesen bekannten Implantatspritzen ist die Innenhülse aus zwei Bauteilen zusammengesetzt, nämlich dem vorderen Nadelschutz und dem hinteren Abstandhalter, die miteinander verrastet sind. Hierzu sind zwei Rasthaken an langen Stegen des Nadelschutzes angeformt, die in Rastfenster des Abstandhalters einschnappen.

Wenn auf diese Rastverbindung Zugkräfte einwirken, werden die Rasthaken gedehnt, wobei bauteilbedingt nur sehr wenig Platz für die Rasthaken verfügbar ist. Bei einem konstruktionsbedingten Querschnitt von vorzugsweise nur ca. 1,5 mm² halten die Rasthaken nur einer Zugkraft von ca. 10 N stand. Wünschenswert ist aber eine höhere Haltekraft der Schnappverbindung und damit eine stabilere Ausbildung der Implantatspritze. Da bauteilbedingt nicht mehr Platz für Rasthaken mit größerem Querschnitt zur Verfügung steht, kommt eine Lösung des Problems durch Rasthaken mit größerem Querschnitt nicht in Betracht, ohne die Handhabbarkeit und das optische Erscheinungsbild der Implantatspritze zu verschlechtern.

Der vorliegenden Erfindung liegt daher die Aufgabe zugrunde, eine bessere Rastverbindung anzugeben, bei der die Haltekraft erheblich erhöht ist.

Diese Aufgabe erfindungsgemäß durch die Merkmale des Patentanspruchs 1 gelöst.

Vorteilhafte Ausgestaltungen der Erfindung sind in den Unteransprüchen gekennzeichnet.

Die Erfindung sieht vor, dass in zwei diametral gegenüber liegenden Laschen des Nadelschutzteils je ein Rastfenster ausgebildet ist, und dass der Abstandhalter an entsprechenden Positionen zwei Rasthaken aufweisen. Beim Verschnappen der beiden Bauteile gleiten die Laschen über die Rasthaken des Abstandhalters, bis die Rasthaken in die Rastfenster der Laschen einrasten. Dabei werden die Endabschnitte der beiden Laschen des Nadelschutzes bündig in Vertiefungen des Abstandhalters aufgenommen. Dabei entsteht eine stabile, unlösbare beidseitige Schnappverbindung, bei der beim Versuch, die beiden Bauteile durch Auseinander-ziehen zu trennen, die eingebrachte Kraft nicht mehr durch Dehnung des Kunststoffs, sondern durch Druck auf den Kunststoff aufgenommen wird. Hierdurch wird die Haltekraft der Verbindung signifikant erhöht, ohne dass der Außenumfang der Innenhülse vergrößert und sein optisches Erscheinungsbild signifikant verändert ist.

In einer vorteilhaften Ausgestaltung der Erfindung haben die beiden Laschen des Nadelhalters, die die Rastfenster aufweisen, eine unterschiedliche Länge, so dass bei entsprechender Ausbildung der beiden Rasthakenbereiche nur eine einzige Montageposition der beiden Bauteile möglich ist.

Die Rasthaken haben bevorzugt eine massive Keilform mit einer in Montagerichtung schräg ansteigender Oberseite und bevorzugt einer im flachen Winkel hinterschnittenen Rückseite, so dass die Schnappverbindung praktisch unlösbar ist. Dabei sollten die Endabschnitte der Laschen bündig in Einsenkungen des Abstandhalters eingreifen.

Weitere Einzelheiten ergeben sich aus der nachfolgenden Beschreibung einer bevorzugten Ausführungsform der Implantatspritze sowie anhand der Zeichnungen. Dabei zeigen:
- Figuren 1a und 1b: verschiedene Darstellungen einer Ausführungsform der Implantatspritze;
- Figuren 2a und 2b: verschiedene Darstellungen der hinteren Hülse mit dem stabförmigen Kolben;

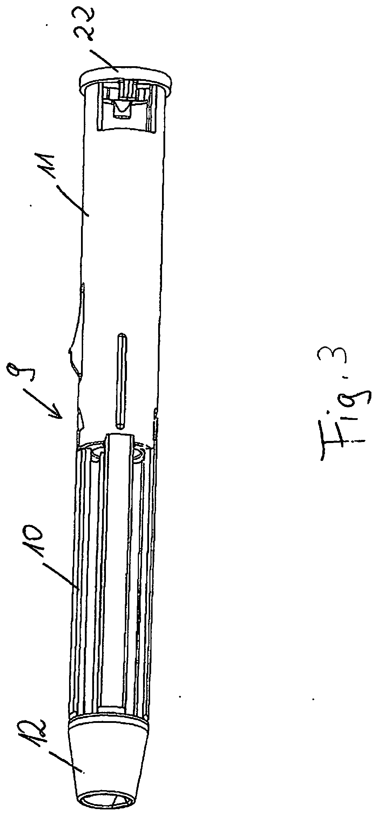

- Figur 3: eine Ansicht der aus Nadelschutzteil und Abstandhalterteil zusammen gesetzten Innenhülse;
- Figur 4: ein herkömmliches Nadelschutzteil der Innenhülse;
- Figur 5: ein Abstandhalterteil der Innenhülse;
- Figur 6: eine erfindungsgemäße Ausführungsform der Innenhülse;
- Figuren 7a und 7b: den Nadelschutz und den Abstandhalter im getrennten Zustand;
- Figur 8: eine Schnittdarstellung des verrasteten Zustands der Innenhülse.

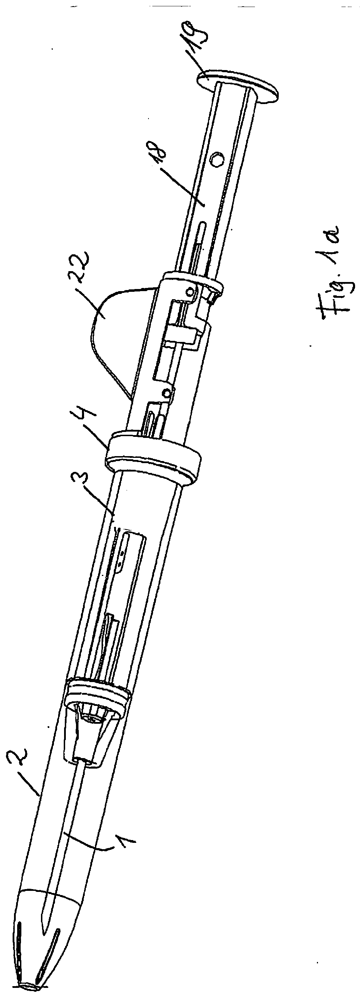

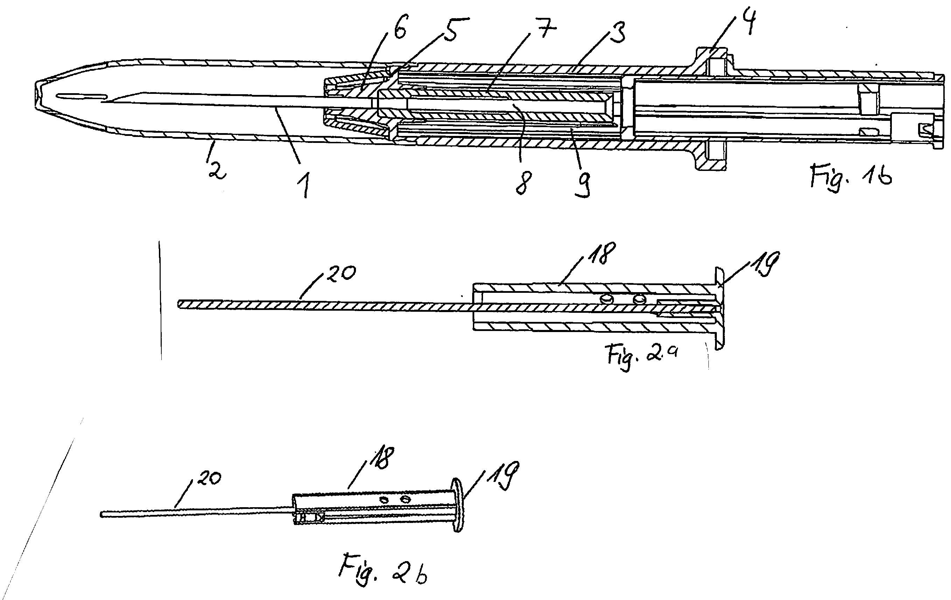

Die Figuren 1a und 1b zeigen einen Ausgangszustand der Implantatspritze, bei dem die Spritzennadel 1 von einer abnehmbaren Schutzkappe 2 überdeckt ist. Die abnehmbare Schutzkappe 2 ist mit einer Außenhülse 3 leicht lösbar verrastet. Die Außenhülse 3 hat eine lang gestreckte zylindrische Form, wobei an ihrem in den Figuren rechten Endabschnitt ein radial überstehender vorderer Griffabschnitt 4 angeformt ist. Die Außenhülse 3 ist über mehrere radial nach Innen verlaufende Stege 5 mit einer Nadelhalterung 6 fest verbunden, in der die Spritzennadel 1 befestigt ist. Axial hinter der Nadelhalterung 6 ist ein Präparataufnahmeteil 7 so angeordnet, dass sein Kanal 8 mit dem Kanal der Spritzennadel 1 fluchtet. In diesem Präparataufnahmeteil 8 befindet sich ein nicht dargestelltes Präparat, das beim Gebrauch der Implantatspritze z.B. in der Bauchdecke eines Patienten abgelegt wird.

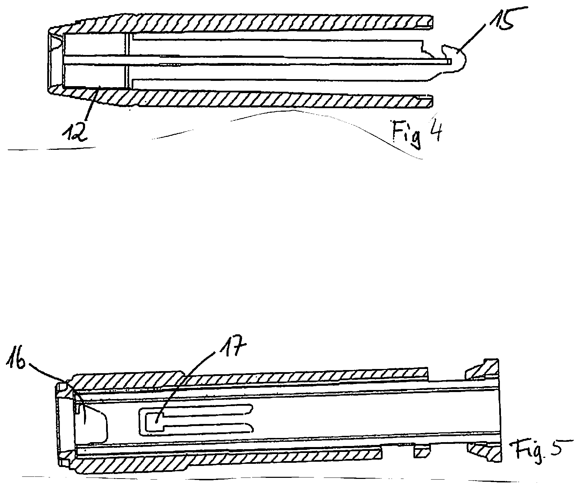

Die Außenhülse 3 sitzt (wenn sie nicht blockiert ist) verschieblich auf einer Innenhülse 9, die aus einem vorderen Nadelschutz 10 und einem hinteren Abstandhalter 11 zusammengesetzt ist. Der Nadelschutz 10 enthält einen vorderen, sich kegelstumpfförmig verengenden Abschnitt 12, der in Umfangsrichtung geschlossen ist und die Spitze der vollständig zurück gezogenen Spritzennadel 1 überdeckt. Der axial anschließende Bereich enthält in Umfangsrichtung beabstandete Stege oder Laschen 13, wobei beim Stand der Technik an den Endabschnitten zweier Stege Haken 15 ausgebildet sind, die in Aussparungen 16 des Abstandhalters 11 der Innenhülse 9 einrasten, um die beiden Teile zu verbinden (Figuren 4 und 5).

Der hintere Teil 11 der Innenhülse 9 enthält einen Federarm 17, der aus der Wand des Innenhülsenteils 11 freigeschnitten ist und mit einem Rasthaken radial nach außen vorsteht. Dieser Federarm 17 mit dem Rasthaken dient als Blockiereinrichtung für die Außenhülse 3, solange eine hintere Hülse 18 nicht in ihre Endstellung in den rückwärtigen Teil 11 der Innenhülse 9 eingeschoben ist.

An dem rückwärtigen Ende der hinteren Hülse 18 ist ein flaches Griffstück 19 angeformt, an dem mittig in der Hülse 18 ein stabförmiger Kolben 20 befestigt ist. Die hintere Hülse 18 enthält einen in axialer Richtung verlaufenden Schlitz, der beim Einschieben der hinteren Hülse 18 in den rückwärtigen Abstandhalter 11 der Innenhülse 9 mittels einer schrägen Fläche so mit dem Federarm 17 zusammenwirkt, dass dessen Rasthaken radial nach innen gezogen wird. Dies ist der Fall, wenn die Innenseite des hinteren Griffstücks 19 an der rückwärtigen Randkante 22 der Innenhülse 9 anschlägt, wobei diese Position leicht lösbar verrastet wird.

Nun kann die Außenhülse 3 zusammen mit der Spritzennadel 1 in ihrer Endposition zurückgezogen werden, in der die beiden Griffstücke 4 und 19 aneinander anliegen. Die Spitze der Spritzennadel 1 befindet sich nun innerhalb des kegelstumpfförmigen Abschnitts 12 der Innenhülse 9.

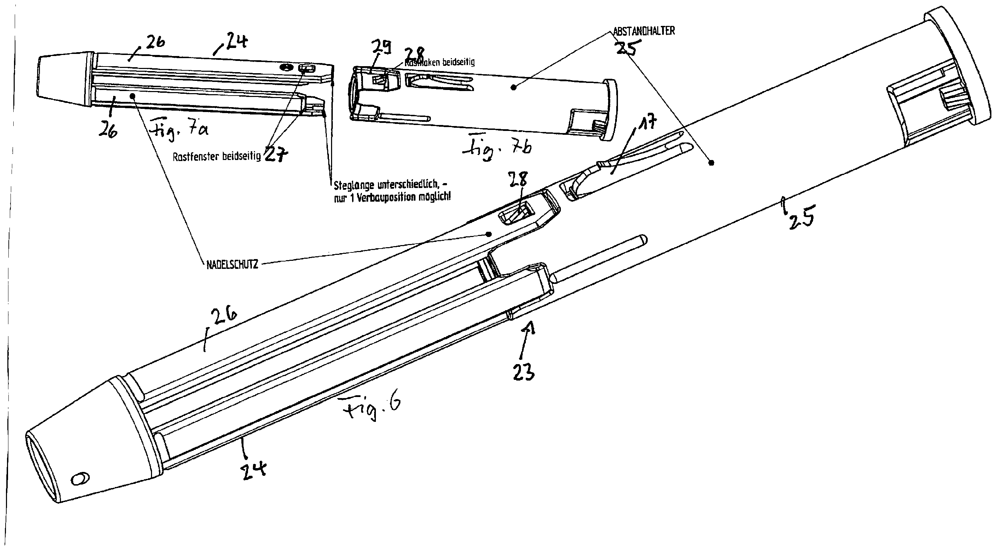

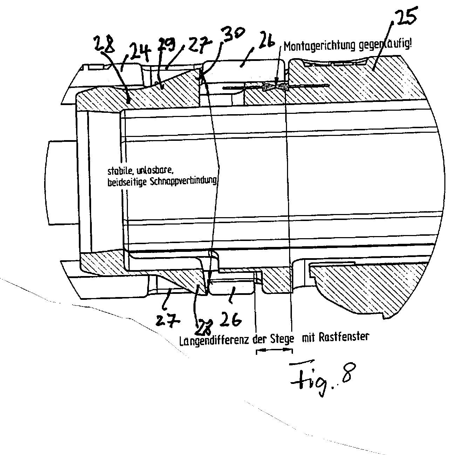

Die Figuren 6 bis 8 zeigen eine Ausführungsform der erfindungsgemäßen Verrastung der Innenhülse 23, die aus dem vorderen Nadelschutz 24 und dem hinteren Abstandhalter 25 zusammengesetzt ist. Der Nadelschutz 24 enthält zwei langgestreckte Laschen 26, in deren Endbereich zwei Rastfenster 27 ausgebildet sind. An dem Abstandhalter 25 sind an entsprechenden Positionen zwei Rasthaken 28 ausgebildet, die beim Zusammenstecken des Nadelschutzes 24 mit dem Abstandhalter 25 in die Rastfenster 27 einschnappen. Die sich mit dem Abstandhalter 25 überlappenden Endbereiche der Laschen 26 treten dabei bündig in Einsenkungen 29 der Abstandhalter 25 ein.

Die Rasthaken 28 haben eine massive Gestalt, die etwa keilförmig ist. Die Oberseite 29 der Rasthaken steigt in Montagerichtung schräg nach oben an, während die Rückseite 30 der Rasthaken 28 in einem flachen Winkel hinterschnitten ist. Hierdurch ist die Schnappverbindung stabil und praktisch unlösbar.

Die beiden Laschen 26 mit den Rastfenstern 27 haben eine unterschiedliche Länge, wobei die beiden zugehörigen Bereiche der Rastfenster entsprechend positioniert sind, so dass nur eine Montageposition möglich ist.

## Patentansprüche

1. Implantatspritze mit einer Kanüle, mit einem Depot-Element zur Aufnahme eines Feststoffimplantats, mit einer Außenhülse mit einem radial überstehenden vorderen Griffabschnitt, mit dem die Kanüle, das Depot-Element und die Außenhülse gemeinsam axial bewegbar sind,
mit einer Innenhülse, auf der die Außenhülse sitzt und in der die Kanüle mit dem Depot-Element verschiebbar ist, wobei die Innenhülse axiale Schlitze aufweist, die radiale Stege der Außenhülse durchgreifen,
ferner mit einem stabförmigen Kolben, der an einem rückwärtigen Griffabschnitt befestigt ist und teilweise von einer hinteren Hülse umgeben ist, die verschieblich in die Innenhülse eingreifen kann,
wobei der stabförmige Kolben durch das Depot-Element in die Kanüle so weit vorschiebbar ist, dass zwischen der Spitze der Kanüle und dem Kopfende des Kolbens ein Abstand verbleibt, der etwa gleich der Länge des abzugebenden Feststoffimplantats ist,
wobei die Außenhülse in der vorgeschobenen Ausgangsstellung bei freiliegender Kanüle durch eine Blockiereinrichtung der Innenhülse lösbar arretiert ist und die Blockiereinrichtung beim Vorschub der hinteren Hülse in ihre Endstellung in die Freigabestellung bewegt wird, wodurch die Außenhülse mittels des vorderen Griffabschnitts auf der Innenhülse zurückziehbar ist, so dass die Kanüle vollständig in die Innenhülse eintritt, und wobei die Innenhülse aus einem vorderen Nadelschutz und einem hinteren Abstandhalter zusammengesetzt ist, die miteinander verrastet sind,
**dadurch gekennzeichnet,**
**dass** in zwei gegenüberliegenden Laschen (26) des Nadelschutzes (24) je ein Rastfenster (27) ausgebildet ist,
**dass** der Abstandshalter (25) an entsprechenden Positionen zwei Rasthaken (28) aufweisen und
**dass** zum Verbinden der beiden Bauteile die Laschen (26) über die Rasthaken (28) des Abstandhalters (25) gleiten, bis die Rasthaken (28) in die Rastfenster (27) einrasten.

2. Implantatspritze nach Anspruch 1,
**dadurch gekennzeichnet,**
**dass** bei der Schnappverbindung die Endabschnitte der Laschen (26) bündig in Einsenkungen (29) des Abstandhalters (25) eingreifen.

3. Implantatspritze nach einem der Ansprüche 1 oder 2,
**dadurch gekennzeichnet,**
**dass** die Rasthaken (28) im wesentlichen eine massive Keilform haben mit einer in Montagerichtung schräg ansteigenden Oberseite (29) und einer im flachen Winkel hinterschnittenen Rückseite (30), so dass die Schnappverbindung unlösbar ist.

4. Implantatspritze nach einem der Ansprüche 1 bis 3,
**dadurch gekennzeichnet,**
**dass** die beiden Laschen (26) eine unterschiedliche Länge haben und die beiden Bereiche der Rastfenster (27) entsprechend ausgebildet sind, so dass nur eine Montageposition möglich ist.

## Claims

1. An implant syringe including a cannula, a storage element for accommodating a solid material implant, an outer sleeve with a radially protruding front gripping section, with which the cannula, the storage element and the outer sleeve are axially movable together, an inner sleeve, on which the outer sleeve sits and in which the cannula is slidable with the storage element, wherein the inner sleeve has axial slits, through which radial webs on the outer sleeve engage, further including a rod-shaped piston, which is fastened to a rear gripping section and is partially surrounded by a rear sleeve, which can movably engage in the inner sleeve, wherein the rod-shaped piston may be advanced through the storage element into the cannula so far that a gap remains between the tip of the cannula and the head end of the piston, which is approximately the same as the length of the solid material implant which is to be delivered, wherein the outer sleeve is releasably locked in the advanced starting position with the cannula exposed by a blocking device and the blocking device is moved into the release position when the rear sleeve is advanced into its end position, whereby the outer sleeve is retractable by means of the front gripping section on the inner sleeve so that the cannula completely enters into the inner sleeve and wherein the inner sleeve is composed of a front needle protector and a rear spacer, which are locked together, **characterised in that** formed in two opposing lugs (26) on the needle protector (24) there is a respective locking window (27), that the spacer (25) has two locking hooks (28) at corresponding positions and that in order to connect the two components the lugs (26) slide over the locking hooks (28) on the spacer (25) until the locking hooks (28) lock into the locking windows (27).

2. An implant syringe as claimed in Claim 1, **characterised in that** in the snap connection the end sections of the lugs (26) engage flush into recesses (29) in the spacer (25).

3. An implant syringe as claimed in one of Claims 1 or 2, **characterised in that** the locking hooks (28) substantially have a massive wedge shape with an upper side (29) rising obliquely in the assembly direction and a rear side (30) undercut at a flat angle so that the snap connection is unreleasable.

4. An implant syringe as claimed in one of Claims 1 to 3, **characterised in that** the two lugs (26) have a different length and the two regions of the locking windows (27) are of corresponding construction so that only one assembly position is possible.

## Revendications

1. Seringue d'implant présentant une canule, un élément de dépôt pour recevoir un implant solide, une gaine extérieure avec une section de préhension avant dépassant radialement, avec laquelle la canule, l'élément de dépôt et la gaine extérieure sont mobiles ensemble axialement,
avec une gaine intérieure sur laquelle repose la gaine extérieure et dans laquelle la canule peut être déplacée avec l'élément de dépôt, dans laquelle la gaine intérieure présente des fentes axiales qui traversent des nervures radiales de la gaine extérieure,
en outre, avec un piston en forme de tige qui est fixé à une section de préhension arrière et qui est partiellement entouré d'une gaine arrière pouvant s'engager de manière coulissante dans la gaine intérieure,
dans laquelle le piston en forme de tige peut être avancé dans la canule à travers l'élément de dépôt jusqu'à laisser entre la pointe de la canule et l'extrémité de la tête du piston une distance environ égale à la longueur de l'implant solide à distribuer,
dans laquelle la gaine extérieure est bloquée de manière détachable dans la position initiale avancée lorsque la canule est exposée par un dispositif de blocage de la gaine intérieure et le dispositif de blocage est déplacé dans la position de libération lors de l'avancement de la gaine arrière dans sa position finale, selon quoi la gaine extérieure peut être rétractée au moyen de la section de préhension avant sur la gaine intérieure de sorte que la canule pénètre complètement dans la gaine intérieure, et dans laquelle la douille intérieure est composée d'une protection d'aiguille avant et d'une entretoise arrière qui sont encliquetées l'une avec l'autre,
**caractérisée en ce**
**qu'**une fenêtre d'encliquetage (27) est respectivement formée dans deux pattes opposées (26) de la protection d'aiguille (24),
**que** l'entretoise (25) présente deux crochets d'encliquetage (28) dans deux positions correspondantes et
**que** pour relier les deux composants, les pattes (26) glissent sur les crochets d'encliquetage (28) de l'entretoise (25) jusqu'à ce que les crochets d'encliquetage (28) s'encliquettent dans les fenêtres d'encliquetage (27).

2. Seringue d'implant selon la revendication 1,
**caractérisée en ce**
**que** lors de l'assemblage par encliquetage, les sections d'extrémité des pattes (26) s'engagent à fleur dans des creux (29) de l'entretoise (25).

3. Seringue d'implant selon l'une quelconque des revendications 1 ou 2,
**caractérisée en ce**
**que** les crochets de verrouillage (28) présentent sensiblement une forme de coin massif avec une face supérieure (29) ascendante obliquement dans le sens de montage et une face arrière (30) contre-dépouillée selon un angle plat de sorte que l'assemblage par encliquetage soit indétachable.

4. Seringue d'implant selon l'une quelconque des revendications 1 à 3,
**caractérisée en ce**
**que** les deux pattes (26) présentent une longueur différente et que les deux zones des fenêtres d'encliquetage (27) sont formées en conséquence, de sorte qu'une seule position de montage soit possible.
